(19) Europäisches Patentamt
European Patent Office
Office européen des brevets

(11) EP 0 722 712 B1

(12) **FASCICULE DE BREVET EUROPEEN**

(45) Date de publication et mention de la délivrance du brevet:
**19.03.1997 Bulletin 1997/12**

(51) Int. Cl.$^6$: **A61K 7/13**

(21) Numéro de dépôt: **96400046.7**

(22) Date de dépôt: **08.01.1996**

(54) **Composition de teinture d'oxydation des fibres kératiniques et procédé de teinture mettant en oeuvre cette composition**

Oxidationsfärbemittel für keratinische Fasern sowie Färbungsverfahren unter Verwendung dieser Zusammensetzung

Keratinous fiber oxidation dyeing composition and the dyeing process using this composition

(84) Etats contractants désignés:
**AT BE CH DE DK ES FR GB GR IE IT LI LU MC NL PT SE**

(30) Priorité: **20.01.1995 FR 9500662**

(43) Date de publication de la demande:
**24.07.1996 Bulletin 1996/30**

(73) Titulaire: **L'OREAL**
**75008 Paris (FR)**

(72) Inventeurs:
- **Audousset, Marie-Pascale**
**F-92600 Asnières (FR)**
- **Cotteret, Jean**
**F-78480 Verneuil sur Seine (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 428 441**
**EP-A- 0 465 340**
**DE-A- 3 031 709**
**DE-A- 3 743 769**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.13.17/3.4

EP 0 722 712 B1

## Description

La présente invention a pour objet une composition prête à l'emploi, pour la teinture d'oxydation des fibres kératiniques, en particulier des fibres kératiniques humaines telles que les cheveux, comprenant, dans un milieu alcalin, un dérivé de paraphénylènediamine convenablement sélectionné, en association avec le 4-hydroxyindole et un agent oxydant, ainsi que le procédé de teinture mettant en oeuvre cette composition. Elle a également pour objet un kit de coloration utilisable pour la préparation d'une telle composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques comme le 4-hydroxyindole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet allemand DE 3 031 709, des compositions pour la teinture d'oxydation des fibres kératiniques renfermant une base d'oxydation telle que la paraphénylènediamine ou le paraaminophénol, et du 4-hydroxyindole à titre de coupleur. De telles compositions ne sont cependant pas entièrement satisfaisantes, notamment au niveau de la tenue des colorations obtenues vis à vis des agents extérieurs et en particulier vis à vis des shampooings.

Il a également déjà été proposé, notamment dans la demande de brevet français FR 2 664 304, des compositions pour la teinture d'oxydation à pH acide des fibres kératiniques renfermant au moins un précurseur de colorant d'oxydation et du 4-hydroxyindole à titre de coupleur. De telles compositions ne sont pas non plus entièrement satisfaisantes notamment au niveau de la sélectivité des colorations obtenues.

Des compositions tinctoriales pour la teinture d'oxydation des cheveux renfermant l'association d'un précurseur de colorant d'oxydation et d'un dérivé substitué du 4-hydroxyindole à titre de coupleur sont également connues par la demande de brevet EP-A-0 428 441.

Or, la demanderesse vient maintenant de découvrir qu'il est possible d'obtenir de nouvelles teintures, qui engendrent des colorations intenses, moins sélectives que les colorations de l'art antérieur obtenues à pH acide et résistant mieux, notamment aux shampooings, que les colorations de l'art antérieur obtenues à pH alcalin, en associant:

- au moins un dérivé de paraphénylènediamine convenablement sélectionné et tel que défini ci-après, à titre de base d'oxydation,

- du 4-hydroxyindole, à titre de coupleur,

- au moins un agent oxydant,

le pH de la composition tinctoriale résultante étant supérieur ou égal à 7.

Cette découverte est à la base de la présente invention.

L'invention a donc pour objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les dérivés de paraphénylénediamine de formules (I) et (II) suivantes :

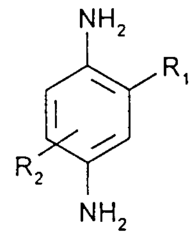

(I)

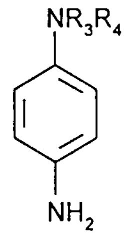

(II)

dans lesquelles:

- $R_1$ représente un radical alkyle en $C_1$-$C_4$ ou monohydroxyalcoxy en $C_2$-$C_4$ ;

- $R_2$ représente un atome d'hydrogène ou un radical alkyle en $C_1$-$C_4$ ;
sous réserve que (i) si $R_2$ représente un radical alkyle en $C_1$-$C_4$, alors $R_1$ est identique à $R_2$, et que (ii) si $R_1$ est en position méta par rapport à $R_2$, alors $R_1$ et $R_2$ ne peuvent désigner simultanément un radical éthyle, et que (iii) si $R_1$ est en position ortho par rapport à $R_2$, alors $R_1$ et $R_2$ ne peuvent désigner simultanément un radical méthyle, et que (iv) si $R_2$ désigne un atome d'hydrogène, alors $R_1$ ne peut désigner un radical méthyle ;

- $R_3$ représente un atome d'hydrogène ou un radical monohydroxyalkyle en $C_1$-$C_4$ ou polyhydroxyalkyle en $C_2$-$C_4$ ;

- $R_4$ représente un radical monohydroxyalkyle en $C_1$-$C_4$, polyhydroxyalkyle en $C_2$-$C_4$ ou alcoxyalkyle en $C_1$-$C_4$,

sous réserve que si $R_4$ représente un radical alcoxyalkyle en $C_1$-$C_4$, alors $R_3$ représente un atome d'hydrogène, et leurs sels d'addition avec un acide,

- du 4-hydroxyindole à titre de coupleur,

- au moins un agent oxydant,

le pH de cette composition prête à l'emploi étant supérieur ou égal à 7.

Les colorations obtenues avec les compositions tinctoriales conformes à l'invention sont moins sélectives que celles de l'art antérieur et présentent par ailleurs une bonne puissance tinctoriale et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale.

Les sels d'addition avec un acide utilisables dans le cadre des compositions tinctoriales de l'invention sont notamment choisis parmi les chlorhydrates, les bromhydrates, les sulfates et les tartrates.

Parmi les dérivés de paraphénylènediamine de formules (I) et (II) ci-dessus, on peut plus particulièrement citer la 2,6-diméthyl paraphénylènediamine, la 2,5-diméthyl paraphénylènediamine, le 1-N-(β-méthoxyéthyl)amino 4-amino benzène, la 2-β-hydroxyéthyloxy paraphénylènediamine, le N,N'-bis-(β-hydroxyéthyl)amino 4-amino benzène, ainsi que leurs sels d'addition avec un acide.

Le ou les dérivés de paraphénylènediamine représentent de préférence de 0,0005 à 10 % en poids environ du poids total de la composition tinctoriale, et encore plus préférentiellement de 0,01 à 5 % en poids environ.

Le 4-hydroxyindole représente de préférence de 0,0001 à 3,5 % en poids environ du poids total de la composition tinctoriale, et encore plus préférentiellement de 0,005 à 1 % en poids environ.

Le pH de la composition tinctoriale telle que définie ci-dessus peut varier entre 7 et 12 et de préférence entre 8 et 11, et peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante :

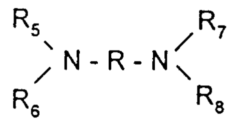

(III)

dans laquelle R est un reste propylène éventuellement substitué par un groupement hydroxyle ou un radical alkyle en $C_1$-$C_4$; $R_5$, $R_6$, $R_7$ et $R_8$, identiques ou différents, représentent un atome d'hydrogène, un radical alkyle en $C_1$-$C_4$ ou hydroxyalkyle en $C_1$-$C_4$.

L'agent oxydant présent dans la composition tinctoriale est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

La composition tinctoriale conforme à l'invention peut encore contenir, en plus des colorants définis ci-dessus, d'autres coupleurs et/ou des colorants directs, notamment pour modifier les nuances ou les enrichir en reflets.

Le milieu approprié pour la teinture (ou support) des compositions tinctoriales est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Les compositions tinctoriales conformes à l'invention peuvent également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

Les compositions tinctoriales conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, et on laisse poser pendant 3 à 40 minutes environ, de préférence 5 à 30 minutes environ, après quoi on rince, on lave éventuellement au shampooing, on rince à nouveau et on sèche.

Selon une forme de réalisation préférée, le procédé comporte une étape préliminaire consistant à stocker sous forme séparée, d'une part, une composition (A) comprenant, dans un milieu approprié pour la teinture, au moins un dérivé de paraphénylènediamine conforme à l'invention et du 4-hydroxyindole et, d'autre part, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques, le pH des compositions (A) et (B) étant tel qu'après mélange de 10 à 90 % de la composition (A) avec 90 à 10 % de la composition (B), le pH du mélange résultant soit supérieur ou égal à 7.

La pH des compositions (A) et (B) peut être ajusté à la valeur désirée au moyen d'agents alcalinisants ou acidifiants classiques et tels que définis précédemment.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition oxydante (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## EXEMPLES

### EXEMPLES 1 ET 2

On a préparé les compositions 1 (A) et 2 (A), conformes à l'invention, suivantes (teneurs en grammes) :

| COMPOSITION | 1 (A) | 2 (A) |
|---|---|---|
| Dichlorhydrate de 2,6-diméthyl paraphénylènediamine | 0,4 | |
| Sulfate de N,N'-bis-(β-hydroxyéthyl)amino 4-amino benzène | | 0,3 |
| 4-hydroxyindole | 0,6 | 0,3 |
| Support de teinture commun (*) | (*) | (*) |
| Eau q.s.p. | 100 g | 100 g |

(*) support de teinture commun :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium, à 55 % de M.A. | 3,0 | g M.A. |
| - Alcool oléique | 5,0 | g |
| - Diéthanolamide d'acide oléique | 12,0 | g |
| - Propylèneglycol | 3,5 | g |
| - Alcool éthylique | 7,0 | g |

| | |
|---|---|
| - Dipropylèneglycol | 0,5 g |
| - Monométhyléther de propylèneglycol | 9,0 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de $NH_3$ | 10,0 g |

Au moment de l'emploi, on a mélangé chaque composition 1 (A) et 2 (A) avec une quantité égale d'une composition (B) constituée par une solution d'eau oxygénée à 20 volumes (6 % en poids).

Chaque composition résultante (composition prête à l'emploi conforme à l'invention, de pH $\geq$ 7) a été appliquée pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous :

| EXEMPLE [COMPOSITION] | NUANCE SUR CHEVEUX NATURELS | NUANCE SUR CHEVEUX PERMANENTES |
|---|---|---|
| 1 [1 (A)] | cendré violacé | cendré violacé puissant |
| 2 [2 (A)] | bleu cendré léger | bleu intense |

**EXEMPLES 3 ET 4 COMPARATIFS**

On a préparé les compositions 3 (A) et 4 (A) suivantes :

| Composition 3 (A) ne faisant pas partie de l'invention : | |
|---|---|
| - Paraphénylènediamine ($4.10^{-3}$ mole) | 0,432 g |
| - 4-hydroxyindole | 0,532 g |
| - Support de teinture commun défini aux exemples 1 et 2 | (*) g |
| - Eau q.s.p. | 100 g |

| Composition 4 (A) conforme à l'invention : | |
|---|---|
| - Dichlorhydrate de 2,6-diméthyl paraphénylènediamine ($4.10^{-3}$ mole) | 0,836 g |
| - 4-hydroxyindole | 0,532 g |
| - Support de teinture commun défini aux exemples 1 et 2 | (*) g |
| - Eau q.s.p. | 100 g |

Au moment de l'emploi, on a mélangé chaque composition 3 (A) et 4 (A) avec une quantité égale d'une composition (B) constituée par une solution d'eau oxygénée à 20 volumes (6 % en poids).

Chaque composition résultante présentait un pH ≥ 7 et a été appliquée pendant 30 minutes sur des mèches de cheveux gris naturels à 90 % de blancs. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Les mèches de cheveux ainsi teintes ont ensuite été soumises à un test de résistance aux shampooings (machine Ahiba-Texomat) :

Les mèches de cheveux ont été placées dans un panier que l'on a immergé dans une solution d'un shampooing standard à 37°C. Le panier a été soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on a retiré les mèches que l'on a rincées puis séchées. Les mèches teintes ont été soumises à 3 épreuves de shampooing consécutives.

La couleur des mèches a été ensuite évaluée à nouveau dans le système MUNSELL au moyen d'un colorimètre CM 2002 MINOLTA de façon à déterminer la dégradation des colorations après ces 3 shampooings.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur entre deux mèches est calculée en appliquant la formule de NICKERSON : $\Delta E = 0{,}4\ Co\Delta H + 6\Delta V + 3\ \Delta C$ , telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et Co représente la pureté de la mèche par rapport à laquelle on désire évaluer la différence de couleur.

Les résultats sont donnés dans le tableau ci-dessous :

| EXEMPLE [COMPOSITION] | Couleur des cheveux avant les shampooings | Couleur des cheveux après les shampooings | Dégradation de la couleur | | | |
|---|---|---|---|---|---|---|
| | | | ΔH | ΔV | ΔC | **ΔE** |
| **3 [3 (A)]** | 7,4 P 2,3 / 1,8 | 8,7 P 2,3 / 1,5 | 1,3 | 0 | 0,3 | **1,84** |
| **4 [4 (A)]** | 2,2 P 2,8 / 1,7 | 2,2 P2,8 / 1,6 | 0 | 0 | 0,1 | **0,3** |

Ces résultats montrent que la composition de l'exemple 4 conforme à l'invention conduit à une coloration résistant mieux aux shampooings que la composition de l'exemple 3, telle que décrite par exemple dans la demande de brevet allemand DE 3 031 709, et ne faisant pas partie de l'invention.

**EXEMPLE 5 ET 6 COMPARATIFS**

On a préparé les compositions 5 (A) et 6 (A) suivantes :

| Composition 5 (A) : | |
|---|---|
| - Dichlorhydrate de 2,6-diméthyl paraphénylènediamine | 0,4 g |
| - 4-hydroxyindole | 0,6 g |
| - Support de teinture commun défini aux exemples 1 et 2 | (*) g |
| - Eau q.s.p. | 100 g |

| Composition 6 (A) : | |
|---|---|
| - Dichlorhydrate de 2,6-diméthyl paraphénylènediamine | 0,4 g |
| - 4-hydroxyindole | 0,6 g |
| - Support de teinture commun défini aux exemples 1 et 2 mais dans lequel l'ammoniaque a été remplacé par une quantité suffisante de monoéthanolamine de façon à ajuster le pH à 9,8 | (*) g |
| - Eau q.s.p. | 100 g |

<u>Procédé de teinture pour l'exemple 5 conforme à l'invention :</u>

Au moment de l'emploi, on a mélangé la composition 5 (A) avec une quantité égale d'une composition 5 (B) constituée par une solution d'eau oxygénée à 20 volumes (6 % en poids).

La composition résultante, présentant un pH de 9,8, a été appliquée pendant 30 minutes, d'une part sur des mèches de cheveux gris naturels à 90 % de blancs (mèche n°1 de cheveux non sensibilisés) et d'autre part sur une mèche de ces mêmes cheveux gris à 90 % de blancs mais ayant subi une permanente (mèche n° 2 de cheveux sensibilisés). Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

La différence de couleur entre cheveux naturels (non sensibilisés) et cheveux permanentés (sensibilisés) a été calculée en appliquant la formule de NICKERSON.

<u>Procédé de teinture pour l'exemple 6 ne faisant pas partie de l'invention :</u>

Au moment de l'emploi, on a mélangé la composition 6 (A) avec une quantité égale d'une composition 6 (B) constituée par une solution d'eau oxygénée à 20 volumes (6 % en poids) dont le pH a été ajusté entre 1 et 1,5 avec 2,5 g d'acide orthophosphorique pour 100 g d'eau oxygénée.

La composition résultante, présentant un pH de 6,8, a été appliquée pendant 30 minutes, d'une part sur des mèches de cheveux gris naturels à 90 % de blancs (mèche n°1 de cheveux non sensibilisés) et d'autre part sur une mèche de ces mêmes cheveux gris à 90 % de blancs mais ayant subi une permanente (mèche n° 2 de cheveux sensibilisés). Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

La différence de couleur entre cheveux naturels (non sensibilisés) et cheveux permanentés (sensibilisés) a été calculée en appliquant la formule de NICKERSON.

Les résultats de sélectivité pour les exemples 5 et 6 sont donnés dans le tableau ci-dessous :

| **EXEMPLES [COMPOSITION]** | **Couleur sur cheveux naturels** | **Couleur sur cheveux permanentés** | **Différence de couleur (sélectivité)** | | | |
|---|---|---|---|---|---|---|
| | | | ΔH | ΔV | ΔC | **ΔE** |
| **5 [5 (A)]** | 2,9 P 2,1 / 2,5 | 8,7 PB 0,1 / 5,0 | 4,2 | 2,0 | 2,5 | **23,7** |
| **6 [6 (A)]** | 9,5 PB 1,2 / 4,5 | 0,2 PB 0 / 2,4 | 9,3 | 1,2 | 2,1 | **30,24** |

Ces résultats montrent que la coloration obtenue à pH alcalin selon le procédé conforme à l'invention est moins sélective que la coloration obtenue à pH acide selon le procédé de l'art antérieur tel que décrit par exemple dans la demande de brevet français FR 2 664 304.

**Revendications**

**1.** Composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les dérivés de paraphénylènediamine de formules (I) et (II) suivantes :

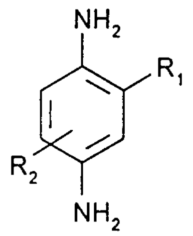

(I)

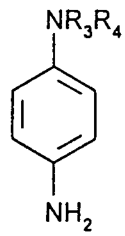

(II)

dans lesquelles :

• $R_1$ représente un radical alkyle en $C_1$-$C_4$ ou monohydroxyalcoxy en $C_2$-$C_4$ ;

• $R_2$ représente un atome d'hydrogène ou un radical alkyle en $C_1$-$C_4$ ; sous réserve que (i) si $R_2$ représente un radical alkyle en $C_1$-$C_4$, alors $R_1$ est identique à $R_2$, et que (ii) si $R_1$ est en position méta par rapport à $R_2$, alors $R_1$ et $R_2$ ne peuvent désigner simultanément un radical éthyle, et que (iii) si $R_1$ est en position ortho par rapport à $R_2$, alors $R_1$ et $R_2$ ne peuvent désigner simultanément un radical méthyle, et que (iv) si $R_2$ désigne un atome d'hydrogène, alors $R_1$ ne peut désigner un radical méthyle ;

• $R_3$ représente un atome d'hydrogène ou un radical monohydroxyalkyle en $C_1$-$C_4$ ou polyhydroxyalkyle en $C_2$-$C_4$ ;

• $R_4$ représente un radical monohydroxyalkyle en $C_1$-$C_4$, polyhydroxyalkyle en $C_2$-$C_4$ ou alcoxyalkyle en $C_1$-$C_4$,

sous réserve que si $R_4$ représente un radical alcoxyalkyle en $C_1$-$C_4$, alors $R_3$ représente un atome d'hydrogène,
et leurs sels d'addition avec un acide,

- du 4-hydroxyindole à titre de coupleur,

- au moins un agent oxydant,

le pH de cette composition prête à l'emploi étant supérieur ou égal à 7.

**2.** Composition selon la revendication 1, caractérisée par le fait que les sels d'addition avec un acide sont choisis parmi les chlorhydrates, les bromhydrates, les sulfates et les tartrates.

**3.** Composition selon la revendication 1 ou 2, caractérisée par le fait que les dérivés de paraphénylènediamine de formules (I) et (II) sont choisis parmi la 2,6-diméthyl paraphénylènediamine, la 2,5-diméthyl paraphénylènediamine, le 1-N-(β-méthoxyéthyl)amino 4-amino benzène, la 2-β-hydroxyéthyloxy paraphénylènediamine, le N,N'-bis-(β-hydroxyéthyl)amino 4-amino benzène, et leurs sels d'addition avec un acide.

**4.** Composition selon l'une quelconque des revendications 1 à 3, caractérisée par le fait que le ou les dérivés de paraphénylènediamine représentent de 0,0005 à 10 % en poids du poids total de la composition tinctoriale.

**5.** Composition selon la revendication 4, caractérisée par le fait que le ou les dérivés de paraphénylènediamine représentent de 0,01 à 5 % en poids du poids total de la composition tinctoriale.

**6.** Composition selon l'une quelconque des revendications 1 à 5, caractérisée par le fait que le 4-hydroxyindole représente de 0,0001 à 3,5 % en poids du poids total de la composition tinctoriale.

7. Composition selon la revendication 6, caractérisée par le fait que le 4-hydroxyindole représente de 0,005 à 1 % en poids du poids total de la composition tinctoriale.

8. Composition selon l'une quelconque des revendications 1 à 7, caractérisée par le fait qu'elle présente un pH compris entre 7 et 12 et de préférence entre 8 et 11.

9. Composition selon l'une quelconque des revendications 1 à 8, caractérisée par le fait que l'agent oxydant est choisi parmi le peroxyde d'hydrogène, le peroxyde d'urée, les bromates de métaux alcalins, les persels tels que les perborates et persulfates.

10. Composition selon l'une quelconque des revendications 1 à 9 caractérisée par le fait qu'elle renferme, en outre, au moins un adjuvant choisi parmi les agents tensio-actifs anioniques, cationiques, non-ioniques, amphotères, zwittérioniques ou leurs mélanges, les polymères anioniques, cationiques, non-ioniques, amphotères, zwittérioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les agents conservateurs, les agents opacifiants.

11. Composition selon l'une quelconque des revendications 1 à 10, caractérisée par le fait qu'elle se présente sous des formes diverses, telles que sous forme de liquides, de crèmes, de gels, ou sous toute autre forme appropriée pour réaliser une teinture des fibres kératiniques, et notamment des cheveux humains.

12. Procédé de teinture des fibres kératiniques et en particulier des fibres kératiniques humaines telles que les cheveux caractérisé par le fait que l'on applique sur ces fibres au moins une composition tinctoriale telle que définie à l'une quelconque des revendications 1 à 11.

13. Procédé selon la revendication 12, caractérisé par le fait qu'il comporte une étape préliminaire consistant à stocker sous forme séparée, d'une part, une composition (A) comprenant, dans un milieu approprié pour la teinture, au moins un dérivé de paraphénylènediamine de formules (I) et/ou (II) tel que défini dans l'une quelconque des revendication 1 à 3 et du 4-hydroxyindole et, d'autre part, une composition (B) renfermant, dans un milieu approprié pour la teinture, au moins un agent oxydant tel que défini dans la revendication 9, et à procéder à leur mélange au moment de l'emploi avant d'appliquer ce mélange sur les fibres kératiniques, le pH des compositions (A) et (B) étant tel qu'après mélange de 10 à 90 % de la composition (A) avec 90 à 10 % de la composition (B), le pH du mélange résultant soit supérieur ou égal à 7.

14. Dispositif à plusieurs compartiments ou "kit" de teinture, caractérisé par le fait qu'un premier compartiment renferme la composition (A) telle que définie dans la revendication 13 et un second compartiment renferme la composition oxydante (B) telle que définie dans la revendication 13.

**Claims**

1. Ready-to-use composition for the oxidation dyeing of keratinous fibres, and especially of human keratinous fibres such as hair, characterized in that it comprises, in a medium appropriate for dyeing:

- at least one oxidation base chosen from para-phenylenediamine derivatives of formulae (I) and (II) below:

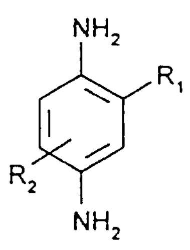

(I)

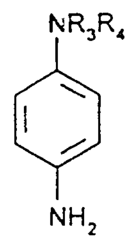

(II)

in which:

• $R_1$ represents a $C_1$-$C_4$ alkyl or $C_2$-$C_4$ monohydroxyalkoxy radical;

• $R_2$ represents a hydrogen atom or a $C_1$-$C_4$ alkyl radical; with the proviso (i) that, if $R_2$ represents a $C_1$-$C_4$ alkyl radical, $R_1$ is identical to $R_2$, and (ii) that, if $R_1$ is in the meta position relative to $R_2$, $R_1$ and $R_2$ cannot simultaneously designate an ethyl radical, and (iii) that, if $R_1$ is in the ortho position relative to $R_2$, $R_1$ and $R_2$ cannot simultaneously designate a methyl radical, and (iv) that, if $R_2$ denotes a hydrogen atom, $R_1$ cannot denote a methyl radical;

• $R_3$ represents a hydrogen atom or a $C_1$-$C_4$ monohydroxyalkyl or $C_2$-$C_4$ polyhydroxyalkyl radical;

• $R_4$ represents a $C_1$-$C_4$ monohydroxyalkyl, $C_2$-$C_4$ polyhydroxyalkyl or $C_1$-$C_4$ alkoxyalkyl radical, with the proviso that, if $R_4$ represents a $C_1$-$C_4$ alkoxyalkyl radical, $R_3$ represents a hydrogen atom, and their addition salts with an acid,

- 4-hydroxyindole as coupler,

- at least one oxidizing agent,

the pH of this ready-to-use composition being greater than or equal to 7.

**2.** Composition according to Claim 1, characterized in that the addition salts with an acid are chosen from hydrochlorides, hydrobromides, sulphates and tartrates.

**3.** Composition according to Claim 1 or 2, characterized in that the para-phenylenediamine derivatives of formulae (I) and (II) are chosen from 2,6-dimethyl-para-phenylenediamine, 2,5-dimethyl-para-phenylenediamine, 1-N-(β-methoxyethyl)amino-4-aminobenzene, 2-β-hydroxyethyloxy-para-phenylenediamine and N,N'-bis(β-hydroxyethyl)amino-4-aminobenzene, and their addition salts with an acid.

**4.** Composition according to any one of Claims 1 to 3, characterized in that the para-phenylenediamine derivative or derivatives represent from 0.0005 to 10 % by weight of the total weight of the dyeing composition.

**5.** Composition according to Claim 4, characterized in that the para-phenylenediamine derivative or derivatives represent from 0.01 to 5 % by weight of the total weight of the dyeing composition.

**6.** Composition according to any one of Claims 1 to 5, characterized in that the 4-hydroxyindole represents from 0.0001 to 3.5 % by weight of the total weight of the dyeing composition.

**7.** Composition according to Claim 6, characterized in that the 4-hydroxyindole represents from 0.005 to 1 % by weight of the total weight of the dyeing composition .

**8.** Composition according to any one of Claims 1 to 7, characterized in that it has a pH of between 7 and 12 and preferably between 8 and 11.

**9.** Composition according to any one of Claims 1 to 8, characterized in that the oxidizing agent is chosen from hydrogen peroxide, urea peroxide, alkali metal bromates, and persalts such as perborates and persulphates.

**10.** Composition according to any one of Claims 1 to 9, characterized in that it additionally comprises at least one adjuvant chosen from anionic, cationic, nonionic, amphoteric or zwitterionic surfactants or mixtures thereof, anionic, cationic, nonionic, amphoteric or zwitterionic polymers or mixtures thereof, inorganic or organic thickeners, antioxidants, penetration agents, sequestering agents, fragrances, buffers, dispersants, conditioners, film-formers, preservatives and opacifying agents.

**11.** Composition according to any one of Claims 1 to 10, characterized in that it is presented in various forms, such as in the form of liquids, creams or gels, or in any other form which is appropriate for dyeing keratinous fibres, and especially human hair.

**12.** Process for dyeing keratinous fibres, especially human keratinous fibres such as hair, characterized in that at least one dyeing composition as defined in any one of Claims 1 to 11 is applied to these fibres.

13. Process according to Claim 12, characterized in that it comprises a preliminary step which consists in storing, separately, on the one hand a composition (A) comprising, in a medium appropriate for dyeing, at least one para-phenylenediamine derivative of formulae (I) and/or (II) as defined in any one of Claims 1 to 3 and 4-hydroxyindole and, on the other hand, a composition (B) comprising, in a medium appropriate for dyeing, at least one oxidizing agent as defined in Claim 9, and in mixing these components at the time of use before applying this mixture to the keratinous fibres, the pH of compositions (A) and (B) being such that, after mixing from 10 to 90 % of the composition (A) with from 90 to 10 % of the composition (B), the pH of the resulting mixture is greater than or equal to 7.

14. Multi-compartment device or kit for dyeing, characterized in that a first compartment contains the composition (A) as defined in Claim 13 and a second compartment contains the oxidizing composition (B) as defined in Claim 13.

**Patentansprüche**

1. Gebrauchsfertige Zusammensetzung zum oxidativen Färben von Keratinfasern und insbesondere von menschlichen Keratinfasern, wie dem Haar,
dadurch gekennzeichnet, daß
sie in einem zum Färben geeigneten Medium

- mindestens eine Oxidationsbase, die unter den p-Phenylendiaminderivaten der folgenden Formeln (I) und (II) und den Additionssalzen dieser Verbindungen mit einer Säure ausgewählt ist:

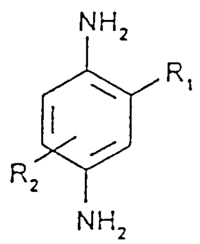 (I)

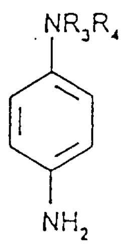 (II)

worin bedeuten:

$R_1$ $C_{1-4}$-Alkyl oder $C_{2-4}$-Monohydroxyalkoxy,

$R_2$ ein Wasserstoffatom oder $C_{1-4}$-Alkyl,
mit der Maßgabe, daß

(i) $R_1$ und $R_2$ identisch sind, wenn $R_2$ eine $C_{1-4}$-Alkylgruppe bedeutet,
(ii) $R_1$ und $R_2$ nicht gleichzeitig Ethyl bedeuten, wenn sich $R_1$ in m-Stellung zu $R_2$ befindet,
(iii) $R_1$ und $R_2$ nicht gleichzeitig Methyl bedeuten, wenn sich $R_1$ in o-Stellung zu $R_2$ befindet, und
(iv) $R_1$ nicht Methyl bedeuten kann, wenn $R_2$ Wasserstoff bedeutet.

$R_3$ Wasserstoff, $C_{1-4}$-Monohydroxyalkyl oder $C_{2-4}$-Polyhydroxyalkyl, und

$R_4$ $C_{1-4}$-Monohydroxyalkyl, $C_{2-4}$-Polyhydroxyalkyl oder $C_{1-4}$-Alkoxyalkyl,
mit der Maßgabe, daß

$R_3$ Wasserstoff bedeutet, wenn $R_4$ $C_{1-4}$-Alkoxyalkyl bedeutet,

- 4-Hydroxyindol als Kuppler und

- mindestens ein Oxidationsmittel,

wobei der pH-Wert der gebrauchsfertigen Zusammensetzung bei 7 oder darüber liegt.

2. Zusammensetzung nach Anspruch 1, dadurch gekennzeichnet, daß die Additionssalze mit einer Säure unter den Hydrochloriden, Hydrobromiden, Sulfaten und Tartraten ausgewählt sind.

3. Zusammensetzung nach Anspruch 1 oder 2, dadurch gekennzeichnet, daß die p-Phenylendiaminderivate der Formeln (I) und (II) unter 2,6-Dimethyl-p-phenylendiamin, 2,5-Dimethyl-p-phenylendiamin, 1-N-(β-Methoxyethyl)amino-4-amino-benzol, 2-β-Hydroxyethyloxy-p-phenylendiamin, N,N'-Bis(β-hydroxyethyl)amino-4-aminobenzol und den Additionssalzen dieser Verbindungen mit einer Säure ausgewählt sind.

4. Zusammensetzung nach einem der Ansprüche 1 bis 3, dadurch gekennzeichnet, daß das oder die p-Phenylendiaminderivate 0,0005 bis 10 Gew.-% des Gesamtgewichts der Färbemittelzusammensetzung ausmachen.

5. Zusammensetzung nach Anspruch 4, dadurch gekennzeichnet, daß das oder die p-Phenylendiaminderivate 0,01 bis 5 Gew.-% des Gesamtgewichts der Färbemittelzusammensetzung ausmachen.

6. Zusammensetzung nach einem der Ansprüche 1 bis 5, dadurch gekennzeichnet, daß das 4-Hydroxyindol 0,0001 bis 3,5 Gew.-% des Gesamtgewichts der Färbemittelzusammensetzung ausmacht.

7. Zusammensetzung nach Anspruch 6, dadurch gekennzeichnet, daß das 4-Hydroxyindol 0,005 bis 1 Gew.-% des Gesamtgewichts der Färbemittelzusammensetzung ausmacht.

8. Zusammensetzung nach einem der Ansprüche 1 bis 7, dadurch gekennzeichnet, daß sie einen pH-Wert im Bereich von 7 bis 12 und vorzugsweise von 8 bis 11 aufweist.

9. Zusammensetzung nach einem der Ansprüche 1 bis 8, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

10. Zusammensetzung nach einem der Ansprüche 1 bis 9, dadurch gekennzeichnet, daß sie ferner mindestens einen Hilfsstoff enthält, der unter den anionischen, kationischen, nichtionischen, amphoteren, zwitterionischen grenzflächenaktiven Stoffen und deren Gemischen, anionischen, kationischen, nichtionischen, amphoteren, zwitterionischen Polymeren und deren Gemischen, anorganischen oder organischen Verdickungsmitteln, Antioxidantien, Penetrationsmitteln, Maskierungsmitteln, Parfums, Puffern, Dispergiermitteln, Konditioniermitteln, Filmbildnern, Konservierungsmitteln und Trübungsmitteln ausgewählt ist.

11. Zusammensetzung nach einem der Ansprüche 1 bis 10, dadurch gekennzeichnet, daß sie in unterschiedlichen Formen vorliegt, wie als Flüssigkeit, Creme, Gel, oder in beliebigen weiteren Formen, die zur Durchführung einer Färbung von Keratinfasern und insbesondere des menschlichen Haares geeignet sind.

12. Verfahren zum Färben von Keratinfasern und insbesondere von menschlichen Keratinfasern, wie dem Haar, dadurch gekennzeichnet, daß auf die Fasern mindestens eine Färbemittelzusammensetzung nach einem der Ansprüche 1 bis 11 aufgetragen wird.

13. Verfahren nach Anspruch 12, dadurch gekennzeichnet, daß es einen vorbereitenden Schritt umfaßt, der darin besteht, getrennt voneinander einerseits eine Zusammensetzung (A), die in einem zum Färben geeigneten Medium mindestens ein p-Phenylendiaminderivat der Formeln (I) und/oder (II) nach einem der Ansprüche 1 bis 3 und 4-Hydroxyindol enthält, und andererseits eine Zusammensetzung (B) aufzubewahren, die in einem zum Färben geeigneten Medium mindestens ein Oxidationsmittel, wie in Anspruch 9 definiert, enthält, und diese bei der Anwendung zu vermischen, bevor das Gemisch auf die Fasern aufgebracht wird, wobei der pH-Wert der Zusammensetzungen (A) und (B) so ist, daß nach dem Mischen von 10 bis 90 % Zusammensetzung (A) und 90 bis 10 % Zusammensetzung (B) der pH-Wert des resultierenden Gemisches bei 7 oder darüber liegt.

14. Vorrichtung mit mehreren Abteilungen oder "Kit" zum Färben, dadurch gekennzeichnet, daß eine erste Abteilung die Zusammensetzung (A) nach Anspruch 13 und eine zweite Abteilung die oxidierende Zusammensetzung (B) nach Anspruch 13 enthält.